# EUROPEAN PATENT APPLICATION

(11) **EP 4 446 401 A1**
(43) Date of publication of application: **16.10.2024**
(21) Application number: 23167825.1
(22) Date of filing: 13.04.2023
(51) Int. Cl.: C12M 1/00, C12M 1/36

(54) **METHOD OF OPERATING AN INTEGRATED BIOPROCESSING SYSTEM TO PERFORM A BIOPROCESS ON A LIQUID IMMUNE OR NAIVE CELL CULTURE**

(71) Applicant: The Automation Partnership (Cambridge) Ltd., Hertfordshire SG8 5WY (GB)
(72) Inventor: Stacey, Adrian, Hertfordshire SG8 5WY (GB); Prouté, Fanny, Hertfordshire SG8 5WY (GB)
(74) Representative: Gottschald Patentanwälte Partnerschaft mbB

(57) **Abstract**

The invention relates to a method of operating an integrated bioprocessing system (1) to perform a bioprocess on a liquid immune or naive cell culture to obtain a processed cell culture, wherein the processed cell culture is destined for autologous or allogenic cell therapy, wherein the integrated bioprocessing systems (1) performs at least one unit operation on the cell culture, wherein the integrated bioprocessing system (1) comprises at least one carrier (5), wherein the carrier (5) carries a, in particular consumable, component and/or substance, wherein the integrated bioprocessing system (1) uses the component or substance to perform the at least one unit operation. It is proposed that the carrier (5) comprises a drive mechanism (8), that the carrier (5) uses the drive mechanism (8) to move itself through the integrated bioprocessing system (1).

## Description

The present invention relates to a method of operating an integrated bioprocessing system to perform a bioprocess on a liquid immune or naive cell culture according to the general part of claim 1, to an integrated bioprocessing system for performing a bioprocess on a liquid immune or naive cell culture according to the general part of claim 14 and to a carrier carrying a preconfigured fluidic structure according to claim 15.

The term "bioprocess" presently represents a biotechnological process, in particular a biotechnological process involving the use of immune cell cultures or naive cell cultures, in particular stem cell cultures. One or more processing steps might be performed on each cell culture. Hence, a bioprocess in this sense might refer to a manufacturing process that involves a sequence of processing steps performed on a cell culture which ultimately will lead to a final product.

Exemplarily, the bioprocess may be in the area of cell and gene therapy, for example to manufacture autologous T cells that are modified to express a chimeric antigen receptor (CAR). These cells might be used for the treatment of various types of hematologic malignancies, including different types of leukemia (blood cancer). Other cell therapies based on naive cells, in particular stem cells and their derivatives are also of interest.

For biotechnological processes involving the use of liquid immune or naive cell cultures the starting material usually is quite heterogeneous regarding its composition, for example as each donor or patient may be in a different condition (e.g., in terms of disease progression, genetic make-up or the immune system history). Therefore, certain steps of the bioprocess need to be flexibly adapted and tailored to each individual cell culture. Furthermore, a bioprocess involving the genetic modification of an immune cell culture will require a sequence of processing steps that is different from the sequence of processing steps required in a bioprocess not involving the genetic modification of an immune cell culture.

Hence, depending on the features of the cell culture and the bioprocess to be performed, various parameters, including for example the type, sequence or configuration of processing steps performed on each immune cell culture will need to be adjusted to the individual features of the cell culture. Further, the cell cultures are typically processed by different devices or need to be transferred between different locations to be processed. This may require an operator to pre-configure or adjust different devices to each cell culture, including the preconfiguration or reconfiguration of single-use components like tubes or tube-sets.

The known prior art, WO 2021/212124 A1, is related to a method according to the general part of claim 1.

This reference discloses an integrated bioprocessing system in which different unit operations are performed on a cell culture. The cell culture and reagents needed are moved between the different devices by a robotic transport mechanism.

An integrated bioprocessing system should be able to flexibly handle different requirements for different cell cultures, different workloads and generally have the possibilities to flexibly configure protocols, functions and devices for new bioprocesses. It is a challenge to improve the flexibility of known systems and related methods. Further, the mechanical failure rate and possible errors should be reduced and at least parts not critical to the cell culture itself should be producible at low cost. A robotic manipulator, while generally providing a high flexibility, needs very high precision and is therefore costly and difficult to program and provides a single point of failure. Integrated bioprocessing systems may comprise carriers for different system components and for moving those system components around, in particular via a manipulator.

The invention is based on the problem of improving the known method such that a further optimization regarding the named challenge is reached.

The above-noted object is solved by the features of the characterizing part of claim 1.

The main realization of the present invention is that carriers moving themselves through the integrated bioprocessing system have many advantages. They provide high flexibility and can move independently of each other such that failures only concern one carrier. The integrated bioprocessing system can be designed more flexibly with separate and possibly nested spaces like shelves and unit operation areas. Further, the integrated bioprocessing system is easier to clean if it uses flat surfaces without further functionality at least at some places. The carriers can be produced cheaper than a manipulator robot as the carriers themselves may allow for a high degree of tolerances.

In detail, it is proposed that the carrier comprises a drive mechanism, that the carrier uses the drive mechanism to move itself through the integrated bioprocessing system.

In an embodiment according to claim 2 the carrier comprises a local control unit for controlling the drive mechanism, further enhancing the autonomous aspect of the carrier. Starting of a movement of the carrier may be controlled centrally by the integrated bioprocessing system.

According to a preferred embodiment described in claim 3, the control unit may determine a position of the carrier in the integrated bioprocessing system, thereby enabling the control unit to make some decisions based on the position. In particular and according to an embodiment of claim 4, the control unit may stop the carrier. Locally evaluating stopping conditions is an effective way to make sure that the carrier stops even if a connection to the central control unit is lost or the integrated bioprocessing system has an error. The movement path of the carrier may be predetermined according to claim 5.

In a preferred embodiment according to claim 6, the predetermined movement path is composed by sections defined independent of the carrier. If the integrated bioprocessing system comprises standard path sections that are composed into the predetermined movement path, the system can be designed in a flexible yet standardized manner and the effort for providing routing is decreased. The integrated bioprocessing system may also comprise sensorial and/or mechanical boundaries and/or guides. According to claim 7, these may be activated and deactivated as needed, providing a further level of safety for the movement of the carriers. It may be possible to always make sure that the right carrier moves along the right movement path and arrives at the correct location.

The integrated bioprocessing system may, according to claim 8, further comprise a transport mechanism. This transport mechanism may allow more complex routing of the carriers than their movement alone. In particular, the transport mechanism may cover a up/down direction (gravity dimension, claim 9).

According to claim 10, the carrier may carry a tray with a fluidic structure. By moving around fluidic structures, a great degree of flexibility of the overall system can be achieved.

Several carriers may meet at a unit operation station to provide different materials needed for the unit operation (claim 11).

Claim 12 described preferred movement paths and claim 13 a preferred mechanical organization of the integrated bioprocessing system.

Another teaching according to claim 14, which is of equal importance, relates to an integrated bioprocessing system for performing a bioprocess on a liquid immune or naive cell culture.

Here, it is essential that the carrier comprises a drive mechanism, that the carrier uses the drive mechanism to move itself through the integrated bioprocessing system.

All explanations given with regard to the proposed method are fully applicable.

Another teaching according to claim 15, which is of equal importance, relates to a carrier carrying a preconfigured fluidic structure for use in the proposed method.

All explanations given with regard to the proposed method and the proposed integrated bioprocessing system are fully applicable.

In the following, embodiments of the invention are explained with respect to the drawing. The drawing shows in
- Fig. 1,: a view of the integrated bioprocessing system,
- Fig. 2,: a possible carrier with a tray and in c) and d) two possible drive mechanisms and
- Fig. 3,: a larger carrier and tray combination.

The integrated bioprocessing system 1 shown in the drawings is preferably adapted to perform a bioprocess for the manufacturing of genetically modified T cells. Here, T cells are genetically modified to express a chimeric antigen receptor (CAR). Consequently, the term "CAR-T cells" describes T cells that have been genetically modified to express a CAR. The genetically modified CAR-T cells, which represent the product of the bioprocess, may be administered to a patient and used to start or resume cancer treatment in the patient. As the bioprocess is performed, the initial immune cell culture is gradually processed. All explanations given are mainly directed to such a bioprocess. It may be pointed out, however, that those explanations are fully applicable to other bioprocesses as well.

The term "liquid immune cell culture" is to be understood in a broad sense and refers to an immune cell culture comprising at least one type of immune cells suspended as particles in any type of liquid. The liquid immune cell culture may comprise other cell types that are not immune cells. Hence, the term "liquid immune cell culture" refers to a liquid immune cell culture at any stage during the bioprocess. Consequently, the type and fraction of immune cells present in the liquid immune cell culture will change during the bioprocess applied as certain immune cells are enriched or depleted from the liquid immune cell culture and/or the immune cells are genetically modified.

The term "immune cells" generally refers to different types of white blood cells. Hence, the term "immune cells" includes a variety of cells, for example, but not limited to dendritic cells, T lymphocytes, also referred to as T cells, B lymphocytes, natural killer cells, macrophages or the like. Immune cells may also include subtypes of immune cells, for example tumor-infiltrating lymphocytes or different types of T cells. Subtypes of a certain type of immune cells may be classified based on the type of antigen present at the cell surface. Hence, the term immune cells may for example refer to T cells comprising the surface antigen CD4 ("CD4+ T cells"). Typically, a certain type of immune cells, e.g., T cells, preferably a certain subtype of immune cells, e.g., CD4+ T cells, will be selectively enriched by the bioprocess, while other immune cells, e.g. macrophages, and/or other cell types that are not immune cells, e.g., erythrocytes, and/or other subtypes of immune cells, e.g., CD8+ T cells, will be depleted from the liquid immune cell culture. The immune cells to be enriched are referred to as target immune cells, all other components to be depleted from the liquid immune cell culture are referred to "impurities". Further, and as mentioned above, the target immune cells might be genetically modified.

The term "naive cells" refers to cells that can still differentiate into different target cell types. In particular, stem cells and their derivatives prior to full differentiation into a specific cell type are naive cells. The term also comprises naive immune cells.

The term "liquid" is to be understood in a broad sense as well and refers to any liquid and/or particle-containing liquid that is processed within the integrated bioprocessing system 1. Hence, the term liquid might refer to media, waste, the liquid immune cell culture, byproducts obtained during the bioprocess, samples and/or an initial immune cell culture.

Preferably, an initial immune cell culture is obtained in a process called "leukapharesis". In leukapharesis, immune cells are obtained from the patient. Additionally or alternatively, the initial immune cell culture might also be obtained from a tissue of the patient. As mentioned above, the initial immune cell culture might also be obtained by one or more donors that are not the patient.

Depending on the source of the initial immune cell culture and the bioprocess to be carried out, the initial immune cell culture, particularly the type, amount and distribution of impurities as well as target immune cells might vary.

Presently, it is preferred that any initial immune cell culture used in the bioprocess is used for only a single patient, in autologous immune therapy the same patient that the cell culture was initially derived from, or a small number of patients, for example up to ten patients. If that is not the case then the bioprocess presently described will yield only a cell culture for a single patient or a small number of patients and cell cultures for other patients are derived from different bioprocesses. Therefore, the proposed integrated bioprocessing system 1 is used for parallelizing small scale bioprocesses. Here and preferably, the bioprocesses are performed according to GMP.

During the performance of bioprocesses, one or more "unit operations" are being performed. Unit operations are basic process steps for reaching a certain physical and/or chemical change of the cell culture. Examples for common unit operations are enrichment, selection, activation and genetic modification.

The integrated bioprocessing system 1 here and preferably performs the bioprocesses by controlling several components of the integrated bioprocessing system 1 to perform several unit operations. The integrated bioprocessing system 1 may comprise a transport mechanism 2 to transport the cell cultures between different stations for the unit operations. The integrated bioprocessing system 1 here and preferably keeps the cell cultures in closed environments, one for each cell culture. The closed environment may be adapted over the course of a bioprocess for example by tube welding. It is then not necessary to sterilize the whole system or use a clean room for the integrated bioprocessing system 1. Here and preferably, the integrated bioprocessing system 1 comprises a first interface 3 at which the cell culture can be entered into the integrated bioprocessing system 1 and/or a second interface 4 at which the cell culture leaves the integrated bioprocessing system 1. The integrated bioprocessing system 1 transports the cell culture from the first interface 3 to at least one, preferably multiple, positions at which unit operations are performed and preferably to the second interface 4 afterwards. Here and preferably, no user intervention is necessary and/or planned and/or performed between the first and the second interface 4 in a normal operation. The integrated bioprocessing system 1 may comprise an enclosure protecting the space functionally between the interfaces from user interventions during a normal operation. The first interface 3 and the second interface 4 may be the same interface.

Proposed is a method of operating an integrated bioprocessing system 1 to perform a bioprocess on a liquid immune or naive cell culture to obtain a processed cell culture. The processed cell culture is destined for autologous or allogenic cell therapy.

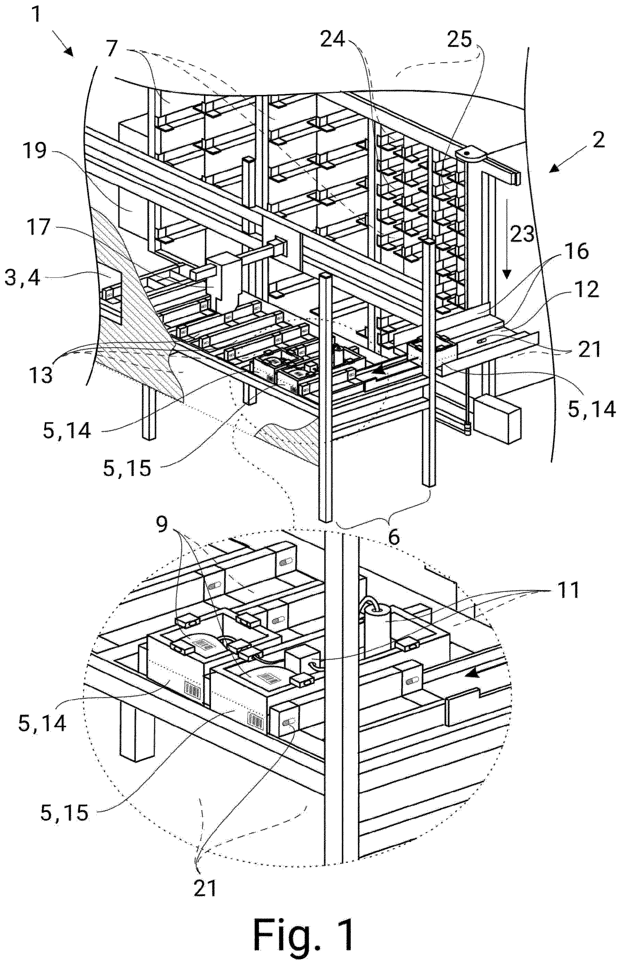

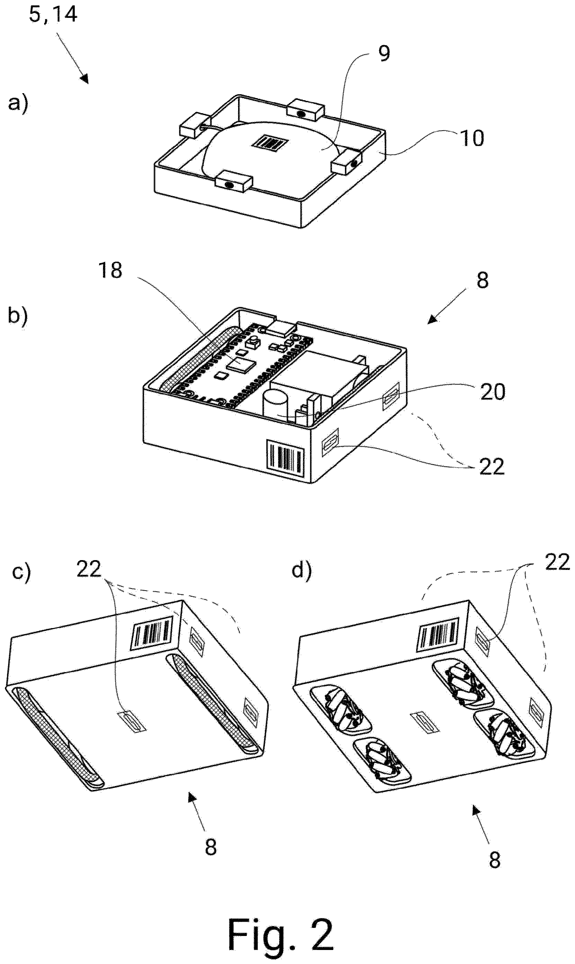

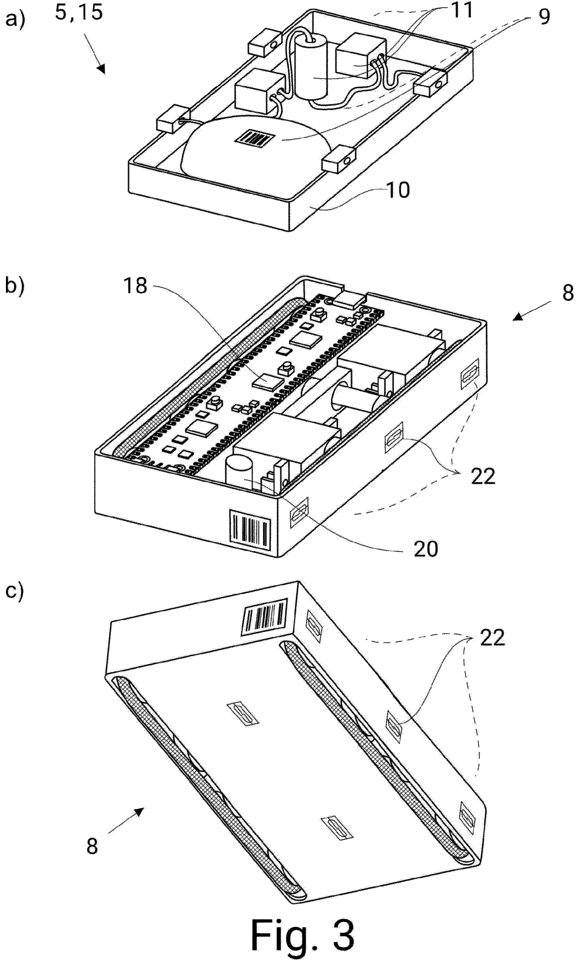

The integrated bioprocessing systems 1 performs at least one unit operation on the cell culture. The integrated bioprocessing system 1 comprises at least one carrier 5. Fig. 1 shows an integrated bioprocessing system 1. In a quick overview, the integrated bioprocessing system 1 comprises a unit operation area 6 at the front and shelves 7 in the back. Between the unit operation area 6 and the shelves 7 the integrated bioprocessing system 1 comprises a transport mechanism 2. Here and preferably, unit operations on the cell culture are mainly or only performed in the unit operation area 6. The shelves 7 are used for storing and possibly for incubating the cell cultures and other media. As can be seen from the design of the shelves 7 and the Figs. 2 and 3, the integrated bioprocessing system 1 may comprise several carriers 5 of different sizes.

The carrier 5 carries a, in particular consumable, component and/or substance, and the integrated bioprocessing system 1 uses the component or substance to perform the at least one unit operation.

Essential is that the carrier 5 comprises a drive mechanism 8 and that the carrier 5 uses the drive mechanism 8 to move itself through the integrated bioprocessing system 1. Here and preferably, the carriers 5 are single components comprising the drive mechanism 8 and a fluidic structure 9 for storing liquid media. However, other embodiments in which the carrier 5 with the drive mechanism 8 only temporarily docks onto a tray 10 with the fluidic structure 9 are also envisioned. Further, it may be the case that other components apart from the fluidic structure 9, for example functional devices 11 of the integrated bioprocessing system 1 are transported via the carrier 5.

In a preferred embodiment, the carrier 5 comprises a reusable part, and a single use part which comprises the fluidic structure 9. The reusable part may comprise the drive mechanism 8, preferably the tray 10 and potentially other active mechanisms e.g. for monitoring and/or pumping. Here and preferably, outside the integrated bioprocessing system 1, the single use part is connected to the reusable part, in particular loaded onto the tray 10. The combination is then loaded onto the integrated bioprocessing system where it may act autonomously or semi-autonomously as described. Once the work for the single use part is completed, for example, because its involvement with a given patient's material is completed, the combination of carrier 5 and tray 10 may be ejected from the integrated bioprocessing system 1. The single use part can then be disposed or taken for further analysis, and the reusable part may then be recycled for use with a subsequent single use part.

Exemplarily, a carrier 5 stored in one of the shelves 7 may carry the cell culture in a fluidic structure 9. The transport mechanism 2, which here and preferably may comprise an extension plate 12 for the shelf 7 onto which the carrier 5 can move itself and which here and preferably may move in two dimensions, may be positioned in front of the shelf 7 such that the carrier 5 can move itself onto the extension plate 12. The carrier 5 may move itself forward in the shelf 7 and onto the extension plate 12. The transport mechanism 2 may then be moved into the position of Fig. 1 such that the carrier 5 can move itself from the extension plate 12 onto the unit operation area 6. The unit operation area 6 may have several slices 13 guiding the carrier 5 into the correct position and the transport mechanism 2 may be placed in front of the correct slice 13.

Here, the carrier 5 carrying the cell culture may be the smaller carrier 14 on the left side of the unit operation area 6 in Fig. 1 and also shown in Fig. 2. On the right side, a further, larger carrier 15 is placed. The larger carrier 15 may have come to its position in the same manner via driving itself and through the transport mechanism 2, which is preferably adapted to handle carriers 5 of different sizes. In Fig. 1, it can be seen that the extension plate 12 may have guide rails 16 in two or more sizes, here in two widths.

The integrated bioprocessing system 1 may comprise a tube welding mechanism 17, here and preferably covering the unit operation area 6. Alternatively or additionally, the integrated bioprocessing system 1 may comprise any other mechanism for connecting fluidic structures 9, in particular via sterile connectors. The smaller carrier 14 and the larger carrier 15 comprise fluidic structures 9. Tubes of the carriers 5 are connected via tube welding and the cell culture is transferred through the tube connection into the larger carrier 15. A unit operation is performed via at least one functional device 11 of the larger carrier 15 on the cell culture. Afterwards, the cell culture may be transferred via another tube welding connection into another smaller carrier 14 or back into the first smaller carrier 14 and sent to a shelf 7 for storage, for example.

Generally, it may be the case that the integrated bioprocessing system 1 comprises a locking mechanism for locking the carrier 5 in place. The locking mechanism may be independent of the carrier 5 or a part of the locking mechanism may be comprised by the carrier 5. Here and preferably, the carrier 5 may be locked in place at a position at which a unit operation is performed, in particular performed in the fluidic structure 9 carried by the carrier 5. The locking mechanism may comprise a form fit blocking the carrier 5 and/or a magnetic mechanism holding the carrier 5. The locking mechanism may comprise a clamp blocking the carrier 5.

Independently of this example, it is generally the case that one or more carriers 5 may carry a fluidic structure 9 with a functional device 11. The functional device 11 may be a pump, a pump head, a centrifuge chamber, a filter, a sorting element, in particular charged plates, an acoustics element for separation, or the like which is necessary for performing a unit operation. Here and preferably the functional device 11 receives energy, in particular mechanical energy, from the integrated bioprocessing system 1, for example via an interface of the unit operation area 6. The functional device 11 may for example be a pump head driven by the integrated bioprocessing system 1. It is to be understood that in this case the energy comes from an element of the integrated bioprocessing system 1 which is not the carrier 5 itself.

Figs. 2 and 3 show embodiments of carriers 5. A carrier 5 may comprise a motor and a revolving element, like a belt, a wheel or a mecanum drive wheel. The revolving element may interact with a counter element of the integrated bioprocessing system 1 such that the interaction guides the carrier 5. For example, the revolving element may be a cog and the counter element a serrated track. In one embodiment, the revolving element does not contact the integrated bioprocessing system 1. Preferably, the revolving element is fully integrated into a housing of the carrier 5. The carrier 5 may then drive itself by magnetic force, for example similar to a suspension railway. Hiding the revolving element greatly increases cleanability of the carrier 5.

As can be seen, further electronic components may be present in a bottom part of the carrier 5. Here and preferably, the carrier 5 comprises a tray 10 for carrying the fluidic structure 9. The tray 10 separates the fluidic structure 9 from the drive mechanism 8. The tray 10 may be removable from the rest of the carrier 5, however, here and preferably, the tray 10 is not removed automatically inside the integrated bioprocessing system 1 but rather outside for preconfiguration of the fluidic structure 9 by an operator. The tray 10 may provide a compartment for the fluidic structure 9 that is configured to contain liquid in case of a leakage of the fluidic structure 9, thereby preventing a spilling of the liquid onto other surfaces of the integrated bioprocessing system 1.

The carrier 5 may comprise a control unit 18. The control unit 18 may control the drive mechanism 8, in particular activating and deactivating the drive mechanism 8 and setting a velocity. Here and preferably, the control unit 18 receives a start signal from a central control unit 19 of the integrated bioprocessing system 1 and starts moving the carrier 5 in reaction to the start signal. The carrier 5 may also comprise a battery 20 for the control unit 18 and/or the drive. The battery 20 may be charged in a storage location, in particular in a shelf 7, and/or at a unit operation location, in particular in the unit operation area 6. The central control unit 19 may be in charge of controlling the integrated bioprocessing system 1, planning movement paths for the transport mechanism 2 and/or the tube welding mechanism 17 and/or the carrier 5 and for initiating the movement of the carrier 5 via the start signal.

Here and preferably, the control unit 18 receives and/or determines a position of the carrier 5 relative to at least one other component of the integrated bioprocessing system 1. It may be the case that the integrated bioprocessing system 1 comprises elements detectable by the carrier 5 at different locations such that the carrier 5 can orient itself by detecting the elements. Here and preferably, the elements are magnets 21 and the carrier 5 comprises a reed sensor 22. Fig. 1 shows several magnets 21 distributed around the integrated bioprocessing system 1.

It may be the case that the position is an absolute position in the integrated bioprocessing system 1, and/or, that the position is a functional position. A functional position is a position that is alone not enough to determine the global position of the carrier 5 inside the integrated bioprocessing system 1 but enough to determine that the carrier 5 has reached a certain functional position. For example, the carrier 5 can detect that it has reached the extension plate 12, in particular a more or less middle position of the extension plate 12 if it detects a magnet 21 after leaving the shelf 7.

According to one embodiment it is proposed that the control unit 18 determines that a stop condition is met by the carrier 5 and stops moving the carrier 5 in reaction to the stop condition. The stop condition may be reaching a certain position, for example the next magnet 21.

Therefore, the carrier 5 may comprise a sensor 22. The control unit 18 then determines that a stop condition is met by the carrier 5 based on a signal of the sensor 22. Preferably, the sensor 22 detects the position of the carrier 5. Generally, it is preferred that the carrier 5 stops its movement at its destination or prior even if the central control unit 19 and/or other parts of the integrated bioprocessing system 1 are not working properly.

As can be seen in Fig. 1, it can be the case that the carrier 5 is moved along a predetermined movement path between a first position and a second position in the integrated bioprocessing system 1. The carrier 5 moves itself along at least a section of the predetermined movement path. The first position in the movement path described above would be at a shelf 7 or at the unit operation area 6, depending on where the carrier 5 is moving to and from. The predetermined movement path may then comprise moving onto the transport mechanism 2, moving the transport mechanism 2 and moving onto the shelf 7 or unit operation station.

Preferably, the control unit 18 receives a description of the section of the predetermined movement path from the central control unit 19. The description may be complex or as simple as "move forwards to the next magnet 21, wait for the next start signal, then move forward to the next magnet 21."

It is preferred that the predetermined movement path is composed of predetermined path sections defined by the integrated bioprocessing system 1 independently of the respective carrier 5 and used for a multitude of carriers 5. Here, the movement path is composed of predetermined path section for at least the carrier 5, which can move between guide rails 16 of the extension plate 12, inside the shelves 7 and in the slices 13 of the unit operation area 6. The possible movement path sections of the transport mechanism 2 may be predetermined or freely determined each time.

Preferably, the integrated bioprocessing system 1 comprises physical representations of the predetermined path sections, here the guide rails 16, the magnets 21 and other components guiding the carriers 5. The physical representations may comprise mechanical and/or sensorial boundaries and/or mechanical and/or sensorial guides. The predetermined movement path may be composed by some out of all possible path sections depending on the first and second position.

In further embodiments, the integrated bioprocessing system 1 comprises a guide wire which the carrier 5 can detect and follow. For example, a current with a defined frequency of for example 1 kHz may be modulated onto the guide wire, in particular onto sections of the guide wire that are currently guiding a carrier 5. The carrier 5 may then move only as long as it detects the frequency. If the integrated bioprocessing system 1 has an error and stops modulating the frequency, the carrier 5 stops. It is also conceivable that the integrated bioprocessing system 1 comprises extendable stops limiting the possible movement range of the carrier 5.

The communication between the control unit 18 and the central control unit 19 may be implemented via wireless communication, in particular Bluetooth or WLAN. The communication may also, alternatively or additionally, be implemented via near field communication or physical contact of the carrier 5 and a counterpart of the integrated bioprocessing system 1, for example metal rails contacting each other.

According to one embodiment it is proposed, that the integrated bioprocessing system 1 activates and/or deactivates the mechanical and/or sensorial boundaries, for example a physical barrier or a detectable but not physical barrier, and/or mechanical and/or sensorial guides, for example the guide rails 16 or the guide wire, to guide the movement of the carrier 5. It may be the case that the control unit 18 stops the carrier 5 if the carrier 5 does not receive active guidance, for example in case of an error of the integrated bioprocessing system 1.

Here and preferably, the or a stop condition can, in particular always, be detected by the control unit 18 without active guidance from the integrated bioprocessing system 1. For example an end stop may have been extended prior to starting the movement of the carrier 5 or the carrier 5 might stop if a guiding signal is no longer present.

As already mentioned, here and preferably, the integrated bioprocessing system 1 comprises a transport mechanism 2 that transports the carrier 5 through the integrated bioprocessing system 1. Preferably, the transport mechanism 2 transports the carrier 5 along a section, in particular a middle section, of the predetermined movement path. In Fig. 1 it can be seen that the extension plate 12 may comprise a channel in the middle for smaller carriers 14 (Fig. 2) and a level above that for wider carriers 5 (not shown).

According to one embodiment it is proposed, that the transport mechanism 2 transports the carrier 5 along the gravity dimension 23 and preferably along at least one dimension orthogonal to the gravity dimension 23, and/or, that the carrier 5 moves itself orthogonal to the gravity dimension 23 and preferably does not move itself along the gravity dimension 23 and/or moves only in one dimension.

Here and preferably, the carrier 5 carries a tray 10, and the tray 10 carries a fluidic structure 9. The carrier 5 may be able to dock to the tray 10 automatically or may be preconfigured with the tray 10 outside the integrated bioprocessing system 1 and enter and/or leave the integrated bioprocessing system 1 together with the tray 10, in particular through the first interface 3 and/or the second interface 4.

Generally, an operator may feed carriers 5 into the integrated bioprocessing system 1 through the first interface 3 with new cell cultures for further bioprocesses and/or with substances to be used. Additionally, it may be the case that the shelves 7 can be fed from the back, in particular with substances that are not the cell culture.

Preferably, the fluidic structure 9 contains the substance, in particular the cell culture. The unit operation may be performed on the cell culture in the fluidic structure 9 while carried by the tray 10, preferably while carried by the carrier 5. The tray 10 may be preconfigured with the fluidic structure 9 outside the integrated bioprocessing system 1. The tray 10 may enter the integrated bioprocessing system 1 with the carrier 5 and the preconfigured fluidic structure 9 and/or leave the integrated bioprocessing system 1 with the carrier 5 and the preconfigured fluidic structure 9.

The term "tray" is not meant to restrict any physical property of the tray 10. In particular, the tray 10 does not have to be substantially flat or the like.

According to one embodiment already described it is proposed, that the integrated bioprocessing system 1 comprises several, in particular different, carriers 5 and/or trays 10 carrying fluidic structures 9 and substances in the fluidic structures 9, that the integrated bioprocessing system 1 plans movements paths for the different carriers 5 such that at least two carriers 5 meet at a unit operation location, preferably, that at the unit operation location the integrated bioprocessing system 1 connects, in particular by tube welding, the fluidic structures 9 carried by the carriers 5, that at least one substance is transferred from one fluidic structure 9 to the other.

The first position and/or the second position may at least sometimes be at a storage location and/or at the unit operation location and/or at a tube welding location and/or at an incubation location.

It is further preferably the case that the integrated bioprocessing system 1 comprises a shelf for storing 24 the carrier 5 and/or the tray 10 and/or a shelf for incubating 25 the cell culture while carried by the carrier 5 and/or the tray 10. Additionally or alternatively, the integrated bioprocessing system 1 may comprise a unit operation area 6 in which unit operations are performed. Preferably, the transport mechanism 2 transports the carrier 5 between the shelf 7 or shelves 7 and the unit operation area 6.

Another teaching which is of equal importance relates to an integrated bioprocessing system 1 for performing a bioprocess on a liquid immune or naive cell culture to obtain a processed cell culture, wherein the processed cell culture is destined for autologous or allogenic cell therapy, wherein the integrated bioprocessing systems 1 performs at least one unit operation on the cell culture, wherein the integrated bioprocessing system 1 comprises at least one carrier 5, wherein the carrier 5 carries a, in particular consumable, component and/or substance, wherein the integrated bioprocessing system 1 uses the component or substance to perform the at least one unit operation.

Here it is essential that the carrier 5 comprises a drive mechanism 8, that the carrier 5 uses the drive mechanism 8 to move itself through the integrated bioprocessing system 1.

All explanations given with regard to the proposed method are fully applicable.

Another teaching which is of equal importance relates to a carrier 5 carrying a preconfigured fluidic structure 9 for use in the proposed method.

All explanations given with regard to the proposed method and the proposed integrated bioprocessing system 1 are fully applicable.

## Claims

1. Method of operating an integrated bioprocessing system (1) to perform a bioprocess on a liquid immune or naive cell culture to obtain a processed cell culture, wherein the processed cell culture is destined for autologous or allogenic cell therapy, wherein the integrated bioprocessing systems (1) performs at least one unit operation on the cell culture, wherein the integrated bioprocessing system (1) comprises at least one carrier (5), wherein the carrier (5) carries a, in particular consumable, component and/or substance, wherein the integrated bioprocessing system (1) uses the component or substance to perform the at least one unit operation,
**characterized in**
**that** the carrier (5) comprises a drive mechanism (8), that the carrier (5) uses the drive mechanism (8) to move itself through the integrated bioprocessing system (1).

2. Method according to claim 1, **characterized in that** the carrier (5) comprises a control unit (18), that the control unit (18) controls the drive mechanism (8), preferably, that the control unit (18) receives a start signal from a central control unit (19) of the integrated bioprocessing system (1) and starts moving the carrier (5) in reaction to the start signal.

3. Method according to claim 2, **characterized in that** the control unit (18) receives and/or determines a position of the carrier (5) relative to at least one other component of the integrated bioprocessing system (1), preferably, that the position is an absolute position in the integrated bioprocessing system (1), and/or, that the position is a functional position.

4. Method according to claim 2 or 3, **characterized in that** the control unit (18) determines that a stop condition is met by the carrier (5) and stops moving the carrier (5) in reaction to the stop condition, preferably, that the carrier (5) comprises a sensor (22), that the control unit (18) determines that a stop condition is met by the carrier (5) based on a signal of the sensor (22), more preferably, that the sensor (22) detects the position of the carrier (5).

5. Method according to one of the preceding claims, **characterized in that** the carrier (5) is moved along a predetermined movement path between a first position and a second position in the integrated bioprocessing system (1), that the carrier (5) moves itself along at least a section of the predetermined movement path, preferably, that the control unit (18) receives a description of the section of the predetermined movement path from the central control unit (19).

6. Method according to claim 5, **characterized in that** the predetermined movement path is composed of predetermined path sections defined by the integrated bioprocessing system (1) independently of the respective carrier (5) and used for a multitude of carriers (5), preferably, that the integrated bioprocessing system (1) comprises physical representations of the predetermined path sections, more preferably, that the physical representations comprise mechanical and/or sensorial boundaries and/or mechanical and/or sensorial guides.

7. Method according to claim 6, **characterized in that** the integrated bioprocessing system (1) activates and/or deactivates the mechanical and/or sensorial boundaries and/or mechanical and/or sensorial guides to guide the movement of the carrier (5), preferably, that the control unit (18) stops the carrier (5) if the carrier (5) does not receive active guidance, for example in case of an error of the integrated bioprocessing system (1).

8. Method according to one of the preceding claims, **characterized in that** the integrated bioprocessing system (1) comprises a transport mechanism (2), that the transport mechanism (2) transports the carrier (5) through the integrated bioprocessing system (1), preferably, that the transport mechanism (2) transports the carrier (5) along a section, in particular a middle section, of the predetermined movement path.

9. Method according one of the preceding claims, **characterized in that** the transport mechanism (2) transports the carrier (5) along the gravity dimension (23) and preferably along at least one dimension orthogonal to the gravity dimension (23), and/or, that the carrier (5) moves itself orthogonal to the gravity dimension (23) and preferably does not move itself along the gravity dimension (23) and/or moves only in one dimension.

10. Method according to one of the preceding claims, **characterized in that** the carrier (5) carries a tray (10), that the tray (10) carries a fluidic structure (9), preferably, that the fluidic structure (9) contains the substance, in particular the cell culture, and/or, that the unit operation is performed on the cell culture in the fluidic structure (9) while carried by the tray (10), preferably while carried by the carrier (5), and/or, that the tray (10) is preconfigured with the fluidic structure (9) outside the integrated bioprocessing system (1), more preferably, that the tray (10) enters the integrated bioprocessing system (1) with the carrier (5) and the preconfigured fluidic structure (9) and/or leaves the integrated bioprocessing system (1) with the carrier (5) and the preconfigured fluidic structure (9).

11. Method according to one of the preceding claims, **characterized in that** the integrated bioprocessing system (1) comprises several, in particular different, carriers (5) and/or trays (10) carrying fluidic structures (9) and substances in the fluidic structures (9), that the integrated bioprocessing system (1) plans movements paths for the different carriers (5) such that at least two carriers (5) meet at a unit operation location, preferably, that at the unit operation location the integrated bioprocessing system (1) connects, in particular by tube welding, the fluidic structures (9) carried by the carriers (5), that at least one substance is transferred from one fluidic structure (9) to the other.

12. Method according to one of claims 5 to 11, **characterized in that** the first position and/or the second position is at least sometimes at a storage location and/or at the unit operation location and/or at a tube welding location and/or at an incubation location.

13. Method according to one of the preceding claims, **characterized in that** the integrated bioprocessing system (1) comprises a shelf for storing (24) the carrier (5) and/or the tray (10) and/or a shelf for incubating (25) the cell culture while carried by the carrier (5) and/or the tray (10), and/or, that the integrated bioprocessing system (1) comprises a unit operation area (6) in which unit operations are performed, preferably, that the transport mechanism (2) transports the carrier (5) between the shelf (7) or shelves (7) and the unit operation area (6).

14. Integrated bioprocessing system for performing a bioprocess on a liquid immune or naive cell culture to obtain a processed cell culture, wherein the processed cell culture is destined for autologous or allogenic cell therapy, wherein the integrated bioprocessing systems (1) performs at least one unit operation on the cell culture, wherein the integrated bioprocessing system (1) comprises at least one carrier (5), wherein the carrier (5) carries a, in particular consumable, component and/or substance, wherein the integrated bioprocessing system (1) uses the component or substance to perform the at least one unit operation,
**characterized in**
**that** the carrier (5) comprises a drive mechanism (8), that the carrier (5) uses the drive mechanism (8) to move itself through the integrated bioprocessing system (1).

15. Carrier carrying a preconfigured fluidic structure (9) for use in the method according to one of claims 1 to 13.
